# EUROPEAN PATENT SPECIFICATION

(11) **EP 2 911 749 B1**
(45) Date of publication and mention of the grant of the patent: **23.01.2019**
(21) Application number: 13785394.1
(22) Date of filing: 23.10.2013
(51) Int. Cl.: A61Q 5/04, A61Q 5/12, A61K 8/81

(54) **COMPOSITION AND PROCESS FOR PERMANENT SHAPING OF HUMAN HAIR**
ZUSAMMENSETZUNG UND VERFAHREN ZUR PERMANENTEN VERFORMUNG MENSCHLICHER HAARE
COMPOSITION ET PROCÉDÉ POUR PERMANENTER DES CHEVEUX HUMAINS

(30) Priority: 25.10.2012 EP 12189990
(43) Date of publication of application: 02.09.2015
(73) Proprietor: Kao Germany GmbH, 64297 Darmstadt (DE)
(72) Inventor: SCHNEIDER, Jörg, 64347 Griesheim (DE); WOOD, Jonathan, 69469 Weinheim (DE)
(86) International application number: PCT/EP2013/072178
(87) International publication number: WO 2014/064156

(56) References cited:
- EP-A1- 2 460 512
- WO-A2-2011/034878
- DE-B- 1 017 333
- US-A1- 2012 164 200
- "Conditioning Polymers", Pure Health magazine, Spring 2011 issue, 1 January 2011 (2011-01-01), pages 22-24, XP055068076, Retrieved from the Internet: URL:http://www.dow.com/personalcare/pdfs/E coSmooth_Silk_PHM_Article.pdf [retrieved on 2013-06-25]
- "Formulating Guidelines for EcoSmooth Silk Conditioning Polymer", , 1 October 2010 (2010-10-01), pages 1-4, XP055068081, Retrieved from the Internet: URL:http://www.dow.com/personalcare/pdfs/E coSmooth_Silk_Formulating_Guidelines.pdf [retrieved on 2013-06-25]

## Description

Present invention relates to a composition and a process for the permanent shaping of human hair used both for the permanent waving of human hair with an excellent waving effect as well as for the straightening of either naturally or chemically curled hair wherein optimum hair conditioning is observed.

It is generally known that permanent waving is carried out in two steps, the reductive splitting of the cysteine disulfide bonds in the hair by a reducing agent and the subsequent neutralization by application of an oxidizing agent, whereby the cysteine disulfide bonds are restored.

The reducing agent still most frequently used today is thioglycolic acid, also in form of the salts thereof, in particular its ammonium salt, although numerous other thio compounds have been proposed for this purpose, which, however, mostly did not succeed.

The compositions containing thioglycolates are customarily applied at a pH-value between 7 and 10, in particular 8.5 and 9.5.

Such compositions vary in their waving and/or straightening performance and, therefore, there is still need for further improvement, especially in permanent shaping of damaged to strongly damaged hair and in particular for permanent shaping hair streaks including parts with various damage level in length and also improvement in hair condition after such carrying our permanent shaping process.

The present invention starts from the task of providing a composition for the permanent shaping of human hair with excellent waving and straightening performance, especially homogeneous curling and straightening efficiency on hair comprising parts with various degree of damage. Hair waved or straightened with composition disclosed herein looks and feels natural upon touching by hand and has improved shine. For waved hair it is especially important that the hair has excellent elasticity and bounce.

Accordingly, the first object of the present invention is an aqueous composition for permanent shaping and/or straightening hair based on at least one reducing agent and comprising further ethylene/octene copolymer dispersed in an aqueous medium comprising ethylene/sodium acrylate copolymer wherein the composition has a pH between 6.5 and 10.5.

Further object of the present invention is the use of an aqueous composition comprising at least one reducing agent and ethylene/octene copolymer dispersed in an aqueous medium comprising ethylene/sodium acrylate copolymer and having pH between 6.5 and 10.5 for permanent shaping and/or straightening hair. Additionally hair is well conditioned with the use in terms of feeling natural upon touching by hand, shine elasticity and bounce.

Further object of the present invention is a process for permanent shaping hair comprising the steps wherein hair is washed or shampooed or made wet and applied an aqueous composition comprising at least one reducing agent and ethylene/octene copolymer dispersed in an aqueous medium comprising ethylene/sodium acrylate copolymer and having pH between 6.5 and 10.5 and processed for 1 to 30 min and rinsed off from hair and subsequently an aqueous composition comprising at least one oxidizing agent is applied and after processing of 1 to 30 min optionally rinsed off from hair.

EP 2460512 discloses compositions for permanent shaping hair comprising dispersed particles of water insoluble polymer. The document does not mention the ethylene/octane copolymer of the present invention.

In the above process, in case the process is carried out for curling of hair, hair is wound on the curlers either after shampooing or washing or wetting or during the application of the reducing agent or after application of the reducing agent. The curlers are taken off from hair after rinsing off the reducing agent or after application of intermediate treatment or before application of the oxidizing agent or after application of the oxidizing agent or at the end of the processing time of oxidizing agent. The time of wounding onto curlers and taking them off is very much dependent on the curling strength aimed. For stronger curls it is preferred that the wounding onto curlers is done before application of the reducing agent and curlers are taken off at the end of the processing time of oxidizing agent.

Ethylene/octene copolymer dispersed in an aqueous medium comprising ethylene/sodium acrylate copolymer is a commercially available raw material under the trade name EcoSmooth Silk from Dow Chemical Company comprising 40 to 44% active matter.

Concentration of ethylene/octene copolymer is in the range of 0.001 to 5%, preferably 0.01 to 3% and more preferably 0.02 to 2% and most preferably 0.05 to 1% by weight calculated to total composition. It should be noted that in case all compositions, i.e. reducing composition and intermediate treatment composition and oxidizing composition comprise ethylene/octene copolymer dispersed in an aqueous medium comprising ethylene/sodium acrylate copolymer the concentration mentioned here is in each composition and calculated to total of each composition.

The permanent shaping compositions according to the invention comprise at least one reducing compound at a concentration of at least 0.5%, preferably at least 1%, more preferably at least 2% and most preferably at least 2.5% by weight calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1.2-propyleneglycol monothioglycolate (see also WO-A 93/1791), 1-3-propanediol monothioglycolate or the isomer mixture resulting therefrom, 1.3-butanediol and 1.4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 0.5 to 15 %, preferably 1.0 to 12.5%, more preferably 2.0 to 12.5%, most preferably 2.5 to 12.5% by weight, calculated to total composition.

The permanent shaping compositions containing reducing agents can, if necessary, comprise alkalizing agents. Their quantity is dependent on the reducing agent and the desired pH-value of the composition. Reducing agent compositions preferably contain 0.1% to 5%, in particular 0.5% to 2.5% by weight thereof, calculated to the total composition. Alkalizing agents preferred within the scope of the invention are ammonium carbamate, ammonia and/or ammonium(bi)carbonate, monoethanolamine and triethanolamine. It is desirable to adjust the pH-value between 6.5 and 10.5, preferably 7 to 9.5.

Permanent shaping compositions according to the invention are suited for use both for the permanent waving, i.e. curling of human hair and for the straightening, i.e. smoothing thereof.

The viscosity best suited for the permanent shaping compositions according to the invention proved to be in the range of 1 to 10,000 mPa.s, preferably about 1 to about 5,000 mPa.s, measured at 20°C in a Brookfield viscosimeter (no. 5 spindle), whereas the viscosity suited for the straightening compositions is preferably higher in a range up to 50,000 mPa.s, preferably up to 30,000 mPa.s measured at 20°C in a Brookfield viscosimeter (no. 5 spindle).

The viscosity is adjusted by addition of the appropriate amounts of thickening agents known **per se,** such as cellulose derivatives. Thickening may as well be realized by formulating a composition in form of an emulsion with the use of C₁₀-C₂₂-fatty alcohols, in mixture with long mono alkyl chain quaternary ammonium surfactants.

Permanent shaping compositions according to the present invention preferably comprise surfactants selected from anionic, nonionic, cationic and amphoteric ones. Their proportion ranges from 0.05 % to 10%, in particular from 0.1 % to 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, permanent shaping compositions comprise at least one cationic surfactant according to general formula 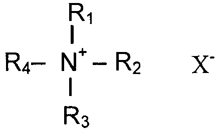 where R₁s a saturated or unsaturated, branched or non-branched alkyl chain with 8-24 C atoms or

R₅CO NH (CH₂)ₙ

where R₅ is saturated or unsaturated, branched or non-branched alkyl chain with 7-21 C atoms and n has typical value of 1 - 4 or

R₆CO O (CH₂)ₙ

where R₆ is saturated or unsaturated, branched or non-branched alkyl chain with 7-21 C atoms and n has typical value of 1 - 4, and
R₂ is a saturated or unsaturated, branched or non-branched alkyl chain with 1-24 C atoms or

R₅CO NH (CH₂)ₙ

where R₅ is saturated or unsaturated, branched or non-branched alkyl chain with 7-21 C atoms and n has typical value of 1 - 4 or

R₆ CO O (CH₂)ₙ

where R₆ is saturated or unsaturated, branched or non-branched alkyl chain with 7-21 C atoms and n has typical value of 1 - 4,
and R₃ and R₄ are same or different lower alkyl chain with 1 to 4 carbon atoms which may be substituted with one or two hydroxyl group, and X is chloride, bromide or methosulfate.

Concantration of cationic surfactant is in the range from 0.05 % to 5 %, preferably 0.1% to 2.5 % by weight, calculated to total composition.

Suitable long-chain quaternary ammonium compounds are in particular cetyl trimethyl ammonium chloride, dimethyl dicetyl ammonium chloride, trimethyl cetyl ammonium bromide chloride, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, lauryl trimethyl ammonium chloride, tris-(oligooxyethyl) alkyl ammonium phosphate, cetyl pyridinium chloride, etc.

Further, permanent shaping compositions of the present invention may comprise additional cationic polymer. Basically suitable are all cationic polymers listed under the generic name "Polyquaternium" in the CTFA International Cosmetic Ingredient Dictionary. Examples are Polyquaternium 4, Polyquaternium 5, Polyquaternium 6, Polyquaternium 7, Polyquaternium 10, Polyquaternium 11, Polyquaternium 16, Polyquaternium 22 and Polyquaternium 28, Polyquaternium 2, Polyquaternium 87, and Polyquaternium 39.

The cationic polymers also include the quaternized products of graft polymers from organopolysiloxanes and polyethyl oxazolines described in EP-A 524 612 and EP-A 640 643. Such polymer is known with its CTFA name Polysilicone-9.

Concentration of one or more additional cationic polymers is in the range from 0.05 % to 2.5 %, preferably 0.1% to 1.5 % by weight, calculated to total composition.

Permanent shaping compositions of present invention can comprise additionally at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, phenoxyethanol, propylene carbonate, N-alkyl pyrrolidone, and urea or their mixture preferably in an amount from about 0.1 % to 10 % by weight, calculated to the total composition.

Permanent shaping composition of the present invention may further comprise ceramide type of compound such as cetyl-PG-hydroxyethylpalmitamide.

Further optional ingredient are sterols,especially the phytosterols as preferred hair restructuring agents. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C10 to C22 may be incorporated into the compositions of the present invention at a concentration of preferably 0.01 to 2.5% by weight calculated to total composition.

Another preferred compound in the permanent shaping composition of the present invention is silicone compounds and especially aminated silicones such as amodimethicone available from for example Dow Corning under the brand names Dow Corning 949 Emulsion, Dow Corning 2-8177 Emuslion and Dow Corning 2-8194. Concentration of silicones, especially amodimethicone, is in the range of 0.05 to 2.5%, preferably 0.1 to 1% by weight calculated to total composition.

Additionally, one or more natural oil component may be incorporated into the compositions of the present invention. Suitable are such as olive oil, almond oil, avocado oil, wheatgerm oil, ricinus oil or their mixture. Concentration of these natural oil ingredients should be 0.01 to 2.5%, preferably 0.01.to 1%, more preferably 0.05 to 0.5% by weight, calculated to total composition.

Further additional compounds may be present in the permanent shaping compositions of the present invention is ubichinone of the formula 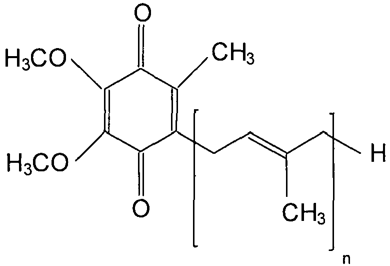 where n is a number between 1 and 10. Preferred ubichinones are the ones where n is a number between 6 and 10 and especially preferred is Ubichinone 50 where n is 10, also known as Coenzyme Q10. Concentration ubichinone of the above formula in permanent shaping compositions of the present invention is from 0.0001 to 1%, preferably from 0.0002 to 0.75%, more preferably from 0.0002 to 0.5% and most preferably from 0.0005 to 0.5% by weight, calculated to total composition.

In another preferred embodiment of the present invention, composition comprises one or more fatty alcohol with a chain length of 12 to 24 C atoms. Concentration of one or more fatty alcohol is in the range of 1 to 15%, preferably 1 to 10%, more preferably 2 to 7.5 and most preferably 2 to 5% by weight calculated to total composition.

Suitable non limiting examples are lauryl alchohol, myristyl alcohol, cetyl alcohol, stearyl alcohol cetearyl alcohol, behenyl alcohol and their mixtures. Preferred are cetyl alcohol, stearyl alcohol cetearyl alcohol, behenyl alcohol and their mixtures. Particularly preferred are cetyl alcohol, stearyl alcohol and their 1 to 1, by weight, mixture cetearyl alcohol.

The compositions used according to the invention can naturally comprise all the substances customarily found in permanent shaping compositions, a list of which will not be given here, and are preferably present as solutions, gels with a higher or lower viscosity, emulsions or creams. They can be single-phase products or compositions packed into separate packaging which are united upon application, as they are disclosed, for example, in DE-C 43 04 828.

In order to avoid repetition, reference is here made to the state of the art as it is described, for example, in "Ullmann's Encyclopedia of Industrial Chemistry", Vol. A12 (1986), pages 588 to 591, and in particular to the monography of K. Schrader, "Grundlagen und Rezepturen der Kosmetika", 2nd. Ed. (1989, Hüthig Buchverlag) pages 823 to 840, as well as the article by D. Hollenberg et. al. in "Seifen-Ole-Fette-Wachse", 117 (1991), pages 81 to 87.

The oxidizing composition may also comprise one or more of the above mentioned ingredients. In any case oxidizing composition comprises at least one oxidizing agent preferably selected from hydrogen peroxide or sodium bromate and preferably at a concentration of 0.5 to 10% by weight calculated to total composition. Oxidizing composition comprising hydrogen peroxide has a pH between 2 and 5, preferably between 2 and 4 and the oxidizing compositions comprising bromate has a pH approximately 6.5 to 7.5.

The intermediate treatment composition comprises at least one inorganic salt and has a pH value between 2.5 to 6, preferably 3 to 5.5 and most preferably 3 to 5. The inorganic salt concentration may very between 1 and 20% and preferably 2 and 15% and more preferably 5 and 15% by weight calculated to total of the composition.

A straightening process may also be carried out in a different process wherein hair is washed and/or shampooed and dried and an aqueous composition according to claims 1 to 5 is applied onto hair and processed for 5 to 45 min and rinsed off from hair and hair is dried the dry hair physically straighten with hot iron at a temperature of 130 to 210°C and subsequently an aqueous oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and optionally rinsed off.

In a further straightening process after physically straightening hair with hot iron at a temperature of 130 to 210°C and prior to application of the oxidizing agent an intermediate treatment composition comprising at least one inorganic salt may be applied onto hair and without rinsing off hair is treated as disclosed above.

The compositions used in permanent shaping process are offered to the users preferably in the form of a kit. Accordingly, further object of the present invention is a kit for permanent shaping hair which comprises at least two compositions kept separately wherein one of the compositions is an aqueous composition according to claims 1 to 5 and the other composition is an aqueous composition and comprises at least one oxidizing agent preferably selected from sodium bromate and hydrogen peroxide.

In a further preferred embodiment of the present invention is that the kit comprises optionally a third composition comprising at least one inorganic salt and optionally comprising ethylene/octene copolymer dispersed in an aqueous medium comprising ethylene/sodium acrylate copolymer.

### Example 1:

| | |
|---|---|
| Ammonium thioglycolate (60%) | 21.3 (% by wt |
| Ammonium hydrogen carbonate | 5.0 |
| 1,3- butylene gylcol | 3.0 |
| EcoSmooth Silk | 1.0 |
| PEG-40-Hydrogenated castor oil | 0.7 |
| Perfume | 0.4 |
| Ammonia, 25% | ad pH 8.3 |
| Water | ad 100.0 |

With this composition the hair was permanently waved for about 15 minutes, rinsed and neutralized for about 8 minutes with a customary 2.5% H₂0₂ composition. Homogeneous wave appearance was obtained which had excellent elasticity and bounce and improved shine and felt natural and soft upon touching. Exclusion of EcoSmooth Silk was resulted in less homogeneous perm appearance.

Additionally EcoSmooth Silk was removed from the reducing composition and was added at the same concentration into the oxidizing composition and hair was permed with a reducing composition without EcoSmooth Silk and an oxidizing composition with EcoSmooth Silk. Here again Homogeneous wave appearance was obtained which had excellent elasticity and bounce and improved shine and felt natural and soft upon touching. It should be noted the results were slightly better and preferred compared to the results obtained when EcoSmooth Silk was comprised in reducing composition.

In a third trial, EcoSmooth Silk was used in both reducing and oxidizing agent whereas the concentrations were halved, i.e. in each composition 0.5% EcoSmooth Silk was used. Homogeneous wave appearance was obtained which had excellent elasticity and bounce and improved shine and felt natural and soft upon touching. When compared the perming efficiency, results obtained with this trial were superior to the previous results described above.

In the above process the use of intermediate treatment according to following composition improved the perming result further.

### Intermediate treatment composition

| | |
|---|---|
| Asparagic acid | 0.25% by weight |
| Glutamic acid | 0.50 |
| Alanin DL | 0.25 |
| Magnesium sulfate | 10.00 |
| Water | q.s. to 100 |

The above composition had a pH of 4.10.

### Example 2:

### Straightening Composition

| | |
|---|---|
| Thioglycolic acid | 8.0 (% by wt.) |
| C₁₆-C₂₂-Fatty alcohol mixture | 3.5 |
| Oleth-50 | 2.5 |
| Laureth-23 | 1.5 |
| EcoSmooth Silk | 2.0 |
| Ethanol | 5.0 |
| Perfume | 0.6 |
| Monoethanolamine | ad pH 9.3 |
| Water | ad 100.0 |

This composition is an effective smoothing composition for kinky hair.

## Claims

1. An aqueous composition for the permanent shaping of human hair, **characterized in that** it comprises at least one reducing agent and ethylene/octene copolymer dispersed in an aqueous medium comprising ethylene/sodium acrylate copolymer, wherein the composition has a pH between 6.5 and 10.5.

2. The composition according to claim 1 **characterized in that** at least one reducing agent is selected from thioglycolic acid, thiolactic acid and their salts, cystein and its hydrochloride salt, and acetylcystein and preferably at a concentration of 0.5 to 15% by weight, calculated to total composition.

3. The composition according to claims 1 and/or 2 **characterized in that** it comprises ethylene/octene copolymer at a concentration in the range of 0.001 to 5% by weight calculated to total composition.

4. Composition according to any of the preceding claims **characterized in that** it comprises at least one cationic polymer.

5. Composition according to any of the preceding claims **characterized in that** it comprises at least one surfactant selected from anionic, nonionic, cationic and amphoteric ones preferably at a concentration of 0.05 to 10% by weight, calculated to total composition.

6. Process for permanent shaping hair **characterized in that** it comprises the steps wherein hair is washed or shampooed or made wet and applied a composition according to claims 1 to 5 and processed for 1 to 30 min and rinsed off from hair and an aqueous composition comprising at least one oxidizing agent is applied and after processing of 1 to 30 min optionally rinsed off from hair.

7. The process according to claim 6 **characterized in that** it comprises additionally the step wherein hair is wound onto curlers
a- after shampooing or washing or wetting, or
b- during the application of the composition according to claims 1 to 5, or
c- after application of the composition according to claims 1 to 5.

8. The process according to claims 6 and 7 **characterized in that** it comprises additionally the step wherein curlers are taken off from hair
a- after rinsing off the composition according to claims 1 to 5, or
b- after application of an aqueous composition comprising at least one oxidizing agent, or
c- at the end of the processing time of oxidizing agent.

9. The process according to claims 6 to 8 **characterized in that** it comprises additionally the step wherein an aqueous composition comprising at least one inorganic salt is applied onto hair after rinsing off the composition according to claims 1 to 5 and before applying oxidizing agent comprising composition and is not rinsed off.

10. The process according to claims 6 to 9 **characterized in that** the aqueous oxidizing composition comprising at least one oxidizing agent comprises additionally ethylene/octene copolymer dispersed in an aqueous medium comprising ethylene/sodium acrylate copolymer.

11. The process according to claims 6 to 10 **characterized in that** the aqueous oxidizing composition comprises at least one oxidizing agent selected from hydrogen peroxide and sodium bromate and preferably comprised at a concentration in the range of 0.5 to 10% by weight calculated to total composition and preferably the composition comprising hydrogen peroxide has a pH between 2 and 5, the composition comprising sodium bromate has a pH between 6.5 and 7.5.

12. Process for straightening hair **characterized in that** it comprises the steps wherein hair is washed and/or shampooed and dried and a composition according to claims 1 to 5 is applied onto hair and processed for 5 to 45 min and rinsed off from hair and hair is dried and the dry hair physically straighten with hot iron at a temperature of 130 to 210°C and hair is applied an aqueous oxidizing composition comprising at least one oxidizing agent, preferably hydrogen peroxide or sodium bromate at a concentration of 0.5 to 10% by weight calculated to total of oxidizing composition, and left on the hair 1 to 20 min and optionally rinsed off.

13. The process according to claim 12 **characterized in that** it comprises additionally application of an aqueous intermediate treatment comprising at least one inorganic salt is applied onto hair after physically straightening hair with hot iron at a temperature of 130 to 210°C and prior to application of the oxidizing agent.

14. Use of a composition according to claims 1 to 5 for permanently shaping and/or straightening hair.

15. Kit for permanently shaping and/or straightening hair comprising at least two compositions kept separately wherein one of the compositions is according to claims 1 to 5 and the other composition comprises at least one oxidizing agent preferably selected from sodium bromate and hydrogen peroxide.

## Patentansprüche

1. Wässrige Zusammensetzung zum dauerhaften Formen von menschlichen Haaren, **dadurch gekennzeichnet, dass** sie mindestens ein Reduktionsmittel und Ethylen/Octen-Copolymer aufweist, dispergiert in einem wässrigen Medium, das Ethylen/Natriumacrylat-Copolymer aufweist, wobei die Zusammensetzung einen pH-Wert von 6,5 bis 10,5 aufweist.

2. Zusammensetzung nach Anspruch 1, **dadurch gekennzeichnet, dass** mindestens ein Reduktionsmittel aus Thioglykolsäure, Thiomilchsäure und deren Salzen, Cystein und dessen Hydrochloridsalz und Acetylcystein ausgewählt ist und vorzugsweise in einer Konzentration von 0,5 Gewichts-% bis 15 Gewichts-% vorliegt, bezogen auf die Gesamtzusammensetzung.

3. Zusammensetzung nach Anspruch 1 und/oder 2, **dadurch gekennzeichnet, dass** sie Ethylen/Octen-Copolymer in einer Konzentration im Bereich von 0,001 Gewichts-% bis 5 Gewichts-% aufweist, bezogen auf die Gesamtzusammensetzung.

4. Zusammensetzung nach einem der vorhergehenden Ansprüche, **dadurch gekennzeichnet, dass** sie mindestens ein kationisches Polymer aufweist.

5. Zusammensetzung nach einem der vorhergehenden Ansprüche, **dadurch gekennzeichnet, dass** sie mindestens ein Tensid aufweist, ausgewählt aus anionischen, nichtionischen, kationischen und amphoteren, vorzugsweise in einer Konzentration von 0,05 Gewichts-% bis 10 Gewichts-%, bezogen auf die Gesamtzusammensetzung.

6. Verfahren zum dauerhaften Formen von Haaren, **dadurch gekennzeichnet, dass** es die Schritte aufweist, wobei die Haare gewaschen oder shampooniert oder nass gemacht werden und eine Zusammensetzung nach Anspruch 1 bis 5 aufgebracht wird und 1 min bis 30 min lang einwirkt und aus den Haaren ausgespült wird und eine wässrige Zusammensetzung, die mindestens ein Oxidationsmittel aufweist, aufgebracht wird und nach einem Einwirken von 1 min bis 30 min gegebenenfalls aus den Haaren ausgespült wird.

7. Verfahren nach Anspruch 6, **dadurch gekennzeichnet, dass** es außerdem den Schritt aufweist, wobei die Haare auf Lockenwickler gezogen werden,
a- nach dem Shampoonieren oder Waschen oder Nassmachen oder
b- während des Aufbringens der Zusammensetzung nach Anspruch 1 bis 5 oder
c- nach dem Aufbringen der Zusammensetzung nach Anspruch 1 bis 5.

8. Verfahren nach Anspruch 6 und 7, **dadurch gekennzeichnet, dass** es außerdem den Schritt aufweist, wobei die Lockenwickler aus den Haaren genommen werden,
a- nach dem Ausspülen der Zusammensetzung nach Anspruch 1 bis 5 oder
b- nach dem Aufbringen einer wässrigen Zusammensetzung, welche mindestens ein Oxidationsmittel aufweist, oder
c- am Ende der Einwirkzeit des Oxidationsmittels.

9. Verfahren nach Anspruch 6 bis 8, **dadurch gekennzeichnet, dass** es außerdem den Schritt aufweist, wobei nach dem Ausspülen der Zusammensetzung nach Anspruch 1 bis 5 und vor dem Aufbringen der Zusammensetzung, die das Oxidationsmittel aufweist, eine wässrige Zusammensetzung, die mindestens ein anorganisches Salz aufweist, auf die Haare aufgebracht wird und nicht ausgespült wird.

10. Verfahren nach Anspruch 6 bis 9, **dadurch gekennzeichnet, dass** die wässrige oxidierende Zusammensetzung, die mindestens ein Oxidationsmittel aufweist, außerdem Ethylen/Octen-Copolymer aufweist, dispergiert in einem wässrigen Medium, das Ethylen/Natriumacrylat-Copolymer aufweist.

11. Verfahren nach Anspruch 6 bis 10, **dadurch gekennzeichnet, dass** die wässrige oxidierende Zusammensetzung mindestens ein Oxidationsmittel aufweist, ausgewählt aus Wasserstoffperoxid und Natriumbromat und vorzugsweise enthalten in einer Konzentration im Bereich von 0,5 Gewichts-% bis 10 Gewichts-%, bezogen auf die Gesamtzusammensetzung, und wobei vorzugsweise die Zusammensetzung, die Wasserstoffperoxid aufweist, einen pH-Wert von 2 bis 5 aufweist, die Zusammensetzung, die Natriumbromat aufweist, einen pH-Wert von 6,5 bis 7,5 aufweist.

12. Verfahren zum Glätten von Haaren, **dadurch gekennzeichnet, dass** es die Schritte aufweist, wobei die Haare gewaschen und/oder shampooniert und getrocknet werden und eine Zusammensetzung nach Anspruch 1 bis 5 auf die Haare aufgebracht wird und 5 min bis 45 min lang einwirkt und aus den Haaren ausgespült wird und die Haare getrocknet werden und die trockenen Haare mit einem Glätteisen bei einer Temperatur von 130 °C bis 210 °C physikalisch geglättet werden und eine wässrige oxidierende Zusammensetzung, die mindestens ein Oxidationsmittel aufweist, vorzugsweise Wasserstoffperoxid oder Natriumbromat in einer Konzentration von 0,5 Gewichts-% bis 10 Gewichts-%, bezogen auf die gesamte oxidierende Zusammensetzung, auf die Haare aufgebracht wird und 1 min bis 20 min lang auf den Haaren belassen wird und gegebenenfalls ausgespült wird.

13. Verfahren nach Anspruch 12, **dadurch gekennzeichnet, dass** es nach dem physikalischen Glätten der Haare mit dem Glätteisen bei einer Temperatur von 130 °C bis 210 °C und vor dem Aufbringen des Oxidationsmittels außerdem Aufbringen einer wässrigen Zwischenbehandlung, die mindestens ein anorganisches Salz aufweist, auf die Haare aufweist.

14. Verwendung einer Zusammensetzung nach Anspruch 1 bis 5 zum dauerhaften Formen und/oder Glätten von Haaren.

15. Kit zum dauerhaften Formen und/oder Glätten von Haaren, aufweisend mindestens zwei Zusammensetzungen, die getrennt gehalten werden, wobei eine der Zusammensetzungen eine nach Anspruch 1 bis 5 ist und die andere Zusammensetzung mindestens ein Oxidationsmittel aufweist, vorzugsweise ausgewählt aus Natriumbromat und Wasserstoffperoxid.

## Revendications

1. Composition aqueuse destinée à la mise en forme permanente des cheveux humains, **caractérisée en ce qu'**elle comprend au moins un agent réducteur et un copolymère éthylène/octène dispersé dans un milieu aqueux comprenant un copolymère éthylène/acrylate de sodium, la composition avant un pH entre 6,5 et 10,5.

2. Composition selon la revendication 1, **caractérisée en ce qu'**au moins un agent réducteur est choisi parmi l'acide thioglycolique, l'acide thiolactique et leurs sels, la cystéine et son sel chlorhydrate, et l'acétylcystéine, et de préférence, à une concentration de 0,5 à 15 % en poids, calculée par rapport à la composition totale.

3. Composition selon les revendications 1 et/ou 2, **caractérisée en ce qu'**elle comprend le copolymère éthylène/octène à une concentration dans la plage de 0,001 à 5 % en poids, calculée par rapport à la composition totale.

4. Composition selon l'une quelconque des revendications précédentes, **caractérisée en ce qu'**elle comprend au moins un polymère cationique.

5. Composition selon l'une quelconque des revendications précédentes, **caractérisée en ce qu'**elle comprend au moins un tensioactif choisi parmi les tensioactifs anioniques, non ioniques, cationiques et amphotères, de préférence à une concentration de 0,05 à 10 % en poids, calculée par rapport à la composition totale.

6. Procédé destiné à la mise en forme permanente des cheveux, **caractérisé en ce qu'**il comprend les étapes dans lesquelles les cheveux sont lavés ou shampouinés, ou mouillés, et sur lesquels est appliquée une composition selon les revendications 1 à 5, et qu'on laisse agir pendant 1 à 30 minutes et qui est éliminée par le rinçage des cheveux, et une composition aqueuse comprenant au moins un agent oxydant est appliquée et après avoir laissé agir pendant 1 à 30 minutes est éventuellement éliminée par rinçage des cheveux.

7. Procédé selon la revendication 6, **caractérisé en ce qu'**il comprend en outre une étape dans laquelle les cheveux sont enroulés autour de bigoudis
a- après le shampooing ou le lavage ou l'humidification, ou
b- au cours de l'application de la composition selon les revendications 1 à 5, ou
c- après l'application de la composition selon les revendications 1 à 5.

8. Procédé selon les revendications 6 et 7, **caractérisé en ce qu'**il comprend en outre une étape dans laquelle les bigoudis sont retirés des cheveux
a- après l'élimination par rinçage de la composition selon les revendications 1 à 5, ou
b- après l'application d'une composition aqueuse comprenant au moins un agent oxydant, ou
c- à la fin de la durée du traitement par l'agent oxydant.

9. Procédé selon les revendications 6 à 8, **caractérisé en ce qu'**il comprend en outre l'étape dans laquelle une composition aqueuse comprenant au moins un sel inorganique est appliquée sur les cheveux après l'élimination par rinçage de la composition selon les revendications 1 à 5 et avant l'application de la composition comprenant l'agent oxydant, et qui n'est pas éliminée par rinçage.

10. Procédé selon les revendications 6 à 9, **caractérisé en ce que** la composition aqueuse oxydante comprenant au moins un agent oxydant comprend en outre du copolymère éthylène/octène dispersé dans un milieu aqueux comprenant le copolymère éthylène/acrylate de sodium.

11. Procédé selon les revendications 6 à 10, **caractérisé en ce que** la composition aqueuse oxydante comprend au moins un agent oxydant choisi parmi le peroxyde d'hydrogène et le bromate de sodium, et qui est de préférence comprise à une concentration dans la plage de 0,5 à 10 % en poids, calculée par rapport à la composition totale, et de préférence, la composition comprenant du peroxyde d'hydrogène a un pH entre 2 et 5, la composition comprenant du bromate de sodium a un pH entre 6,5 et 7,5.

12. Procédé destiné à lisser les cheveux, **caractérisé en ce qu'**il comprend les étapes dans lesquelles les cheveux sont lavés et/ou shampouinés et séchés et une composition selon les revendications 1 à 5 est appliquée sur les cheveux et agit pendant 5 à 45 minutes, et est éliminée des cheveux par rinçage et les cheveux sont séchés, et les cheveux secs sont lissés de manière physique avec un fer chaud à une température entre 130 et 210 °C et sur les cheveux est appliquée une composition aqueuse oxydante comprenant au moins un agent oxydant, de préférence, du peroxyde d'hydrogène ou du bromate de sodium, à une concentration de 0,5 à 10 % en poids, calculée par rapport à la totalité de la composition oxydante, et qui est laissée sur les cheveux pendant 1 à 20 minutes et qui est éventuellement éliminée par rinçage.

13. Procédé selon la revendication 12, **caractérisé en ce qu'**il comprend en outre l'application d'un traitement aqueux intermédiaire comprenant au moins un sel inorganique qui est appliqué sur les cheveux après le lissage des cheveux de manière physique par un fer chaud à une température entre 130 et 210 ° C et préalablement à l'application de l'agent oxydant.

14. Utilisation d'une composition selon les revendications 1 à 5 destinée à la mise en forme permanente et/ou au lissage des cheveux.

15. Kit destinée à la mise en forme permanente et/ou au lissage des cheveux comprenant au moins deux compositions conservées de manière séparée, où l'une des compositions est selon les revendications 1 à 5 et l'autre composition comprend au moins un agent oxydant de préférence choisi parmi le bromate de sodium et le peroxyde d'hydrogène.
